# EUROPÄISCHE PATENTANMELDUNG

(11) **EP 1 862 176 A1**
(43) Veröffentlichungstag der Anmeldung: **05.12.2007**
(21) Anmeldenummer: 06011345.3
(22) Anmeldetag: 31.05.2006
(51) Int. Cl.: A61K 39/39, A61K 47/02, C07F 1/00, A61K 39/29

(54) **Ein Verfahren zur Herstellung einer Impfzusammensetzung**

(71) Anmelder: Rhein Biotech Gesellschaft für neue biotechnologische Prozesse und Produkte mbH, 40595 Düsseldorf (DE)
(72) Erfinder: Fiedler, Bernd Dr., 59427 Unna (DE); Weyhenmeyer, Roland Dr., 51429 Bergisch Gladbach (DE); Melber, Karl Dr., 40591 Düsseldorf (DE); Janowicz, Zbigniew Dr., 40699 Erkrath (DE)
(74) Vertreter: Hakvoort, Ansgar

(57) **Zusammenfassung**

Die vorliegende Erfindung betrifft ein Verfahren zur Herstellung einer Impfstoffzusammensetzung, beinhaltend die Verfahrensschritte:
i) Bereitstellen eines Volumens V₁ einer wässrigen Phase P₁ beinhaltend eine Aluminiumverbindung, deren Oberfläche mit Phosphat-Ionen in Kontakt gebracht worden ist,
ii) Bereitstellen eines Volumens V₂ einer wässrigen Phase P₂ eines Antigens,
iii) Zugabe des Volumens V₂ der wässrigen Phase P₂ zum Volumen V₁ der wässrigen Phase P₁ unter Rühren des Volumens V₁,

wobei mindestens eine, vorzugsweise jede der folgenden Bedingungen erfüllt ist:
a) die Aluminiumverbindung wurde nach dem in Kontakt bringen mit den Phosphat-Ionen und vor dem Verfahrensschritt iii) erhitzt;
b) die wässrige Phase P₁ beinhaltet weniger als 10 mg/ml Aluminium;
c) die wässrige Phase P₂ beinhaltet weniger als 1 mg/ml des Antigens;
d) das Volumen V₂ wird mit einer Geschwindigkeit von höchstens 10 % des Betrages des Volumens V₁/Minute zum Volumen V₁ zugegeben.

Die Erfindung betrifft auch die durch dieses Verfahren erhältliche Impfzusammensetzung, eine Impfstoffzusammensetzung beinhaltend ein Substrat und ein Antigen sowie die Verwendung einer Impfzusammensetzung.

## Beschreibung

Die vorliegende Erfindung betrifft ein Verfahren zur Herstellung einer Impfstoffzusammensetzung, die durch dieses Verfahren erhältliche Impfstoffzusammensetzung, eine Impfstoffzusammensetzung beinhaltend ein Substrat und ein Antigen sowie die Verwendung einer Impfstoffzusammensetzung.

Impfzusammensetzungen zur Prävention viraler Erkrankungen, beispielsweise zur Prävention einer Hepatitis B-Infektion, sind aus dem Stand der Technik bekannt.

So beschreibt EP-A-0 576 478 eine Impfstoffzusammensetzung basierend auf Aluminiumsalzpartikeln, auf deren Oberfläche ein Antigen sowie 3-O-deacetyliertes Monophosphoryl-Lipid A (3D-MPL) absorbiert worden sind. EP-A- 0 689 454 und EP-A-0 633 784 beschreiben ein Verfahren zur Herstellung einer Impfzusammensetzung, bei dem zunächst ein Antigen und anschließend ein 3D-MPL als Adjuvants auf der Oberfläche eines Aluminiumsalzpartikels absorbiert werden. Die in diesen europäischen Patentanmeldungen beschriebenen Impfstoffzusammensetzungen sind dadurch gekennzeichnet, dass das Adjuvants und das Antigen zumindest teilweise auf den gleichen Aluminiumsalzpartikeln immobilisiert sind.

WO-A-00/23105 beschreibt eine Adjuvants-Zusammensetzung umfassend Immunstimulantien, die an der Oberfläche eines Metallsalzpartikels absorbiert sind, wobei das Metallsalzpartikel im Wesentlichen frei von anderen Antigenen ist. Die in diesem Stand der Technik beschriebenen Adjuvants-Zusammensetzungen werden dadurch erhalten, dass zunächst das Adjuvants an der Oberfläche eines Aluminiumsalzpartikels und, getrennt davon, das Antigen auf der Oberfläche eines anderen Aluminiumsalzpartikels immobilisiert wird. Zur Vervollständigung der Impfstoffzusammensetzung werden dann die Aluminiumsalzpartikel miteinander vermischt. Die in dieser internationalen Patentanmeldung beschriebene Impfstoffzusammensetzung ist demnach dadurch gekennzeichnet, dass das Adjuvants und das Antigen auf verschiedenen Aluminiumsalzpartikeln immobilisiert sind.

Der Nachteil der aus dem Stand der Technik bekannten Impfstoffzusammensetzungen besteht unter anderem darin, dass sie durch eine nur schwache Stabilität gekennzeichnet sind. Diese schwache Stabilität der aus den drei Komponenten Aluminiumsalzpartikel, Antigen und Adjuvans bestehenden Impfstoffzusammensetzungen äußert sich unter anderem darin, dass oftmals wenige Minuten nach der Herstellung der Impfstoffzusammensetzungen einzelne Komponenten in den in der Regel flüssigen Zubereitungen sedimentieren.

Weiterhin sind insbesondere diejenigen Impfstoffzusammensetzungen, bei denen Adjuvans und Antigen auf verschiedenen Aluminiumsalzpartikeln immobilisiert sind, wie etwa diejenige Zusammensetzung, die in der WO-A-00/23105 beschrieben ist, eine nur geringe immunstimulatorischen Wirkung. Darüber hinaus erfordert insbesondere dass in der WO-A-00/23105 beschrieben Verfahren den Einsatz von mindestens zwei separaten Rührkesseln, in denen die Aluminiumsalzpartikel-Antigen-Zusammensetzung sowie die Aluminiumsalzpartikel-Adjuvans-Zusammensetzung hergestellt wird.

Ein weiterer Nachteil der aus dem Stand der Technik bekannten Impfstoffzusammensetzungen sind die oftmals starken Schwankungen in der immunstimulatorischen Wirkung der Impfstoffzusammensetzungen zwischen einzelnen Produktionschargen.

Der vorliegenden Erfindung lag die Aufgabe zugrunde, die sich aus dem Stand der Technik ergebenden Nachteile von Impfstoffzusammensetzungen, insbesondere von Impfstoffzusammensetzungen zur Prävention einer Hepatitis B-Erkrankung, zu überwinden.

Insbesondere lag der vorliegenden Erfindung die Aufgabe zugrunde, ein Verfahren zur Herstellung einer Impfstoffzusammensetzung anzugeben, welches die Herstellung einer Impfstoffzusammensetzung mit möglichst konstanter und chargenunabhängiger immunstimulatorischer Wirkung ermöglicht. Dieses Verfahren sollte möglichst mit einem einzigen Rührkessel, in den die einzelnen Komponenten eingebracht werden können, durchführbar sein und trotzdem die Herstellung von Impfstoffzusammensetzung mit einer hohen immunstimulatorischen Wirkung ermöglichen.

Auch lag der vorliegenden Erfindung die Aufgabe zugrunde, ein Verfahren zur Herstellung einer Impfstoffzusammensetzung anzugeben, welches die Herstellung möglichst lagerungsstabiler Impfstoffzusammensetzungen ermöglicht.

Darüber hinaus sollte die durch das erfindungsgemäße Verfahren erhältliche Impfstoffzusammensetzung, insbesondere dann, wenn es sich im eine Impfstoffzusammensetzung zur Prävention einer HBV-Erkrankung handelt, einen ausreichenden Impfschutz möglichst schon nach zwei Verabreichungen der Impfstoffzusammensetzung (= 2-Dosen-Impfstoff) gewährleisten.

Einen Beitrag zur Lösung der eingangs genannten Aufgaben leistet ein Verfahren zur Herstellung einer Impfstoffzusammensetzung, beinhaltend die Verfahrensschritte:
i) Bereitstellen eines Volumens V₁ einer wässrigen Phase P₁ beinhaltend eine Aluminiumverbindung, deren Oberfläche mit Phosphat-Ionen in Kontakt gebracht worden ist,
ii) Bereitstellen eines Volumens V₂ einer wässrigen Phase P₂ eines Antigens,
iii) Zugabe des Volumens V₂ der wässrigen Phase P₂ zum Volumen V₁ der wässrigen Phase P₁ unter Rühren des Volumens V₁,
   wobei mindestens eine, vorzugsweise jede der folgenden Bedingungen erfüllt ist:
   a) die Aluminiumverbindung wurde nach dem in Kontakt bringen mit den Phosphat-Ionen und vor dem Verfahrensschritt iii) erhitzt;
   b) die wässrige Phase P₁ beinhaltet weniger als 10 mg/ml Aluminium;
   c) die wässrige Phase P₂ beinhaltet weniger als 1 mg/ml des Antigens;
   d) das Volumen V₂ wird mit einer Geschwindigkeit von höchstens 10 % des Betrages des Volumens V₁/Minute zum Volumen V₁ zugegeben.

Erfindungsgemäß bevorzugte Ausführungsformen des erfindungsgemäßen Verfahrens sind diejenigen Verfahren, bei denen die folgenden Eigenschaften bzw. Kombinationen von Eigenschaften erfüllt sind: a), b), c), d), a)b), a)c), a)d), b)c), b)d), c)d), a)b)c), a)b)d), a)c)d), b)c)d) und a)b)c)d), wobei a), b)c)d) und a)b)c)d besonders bevorzugt und a)b)c)d) am meisten bevorzugt ist.

Im Verfahrensschritt i) des erfindungsgemäßen Verfahrens wird zunächst ein Volumen V₁ einer wässrige Phase P₁ beinhaltend eine Aluminiumverbindung, deren Oberfläche mit Phosphat-Ionen in Kontakt gebracht worden ist, bereitgestellt. Bei dieser wässrigen Phase P₁ kann es sich, je nach der enthaltenen Aluminiumverbindung, um eine wässrige Lösung, um eine wässrige Dispersion, um eine wässrige Emulsion oder um eine wässrige Suspension handeln. Die Bezeichnung "wässrig", wie sie hierin verwendet wird, soll lediglich angeben, dass die entsprechende Phase Wasser als Lösungsmittel oder als Teil eines Lösungsmittelgemisches enthält.

Bevorzugte Aluminiumverbindungen beinhalten Verbindungen ausgewählt aus der Gruppe bestehend aus Aluminiumsulfat, Aluminiumchlorid, Aluminiumhydroxid, insbesondere Aluminiumorthohydroxide, wie etwa Hydrargillit (γ-Al(OH)₃), Bayerit (α-Al(OH)₃) oder Nordstrandit (β-Al(OH)₃), Aluminiummetahydroxide, wie etwa Böhmit (γ-Al(O)OH), welches kommerziell beispielsweise unter der Bezeichnung Alhydrogel^{®} oder Rehydragel^{®} erhältlich ist, oder Diaspor (α-Al(O)OH), Aluminiumphosphate, wie etwa AlPO₄ oder amorphes Aluminiumhydroxyphosphat-Gel (Al(OH)ₘ(PO4)ₙ), wie es beispielsweise unter der Bezeichnung AdjuPhos^{®} oder RehydraPhos^{®} kommerziell erhältlich ist, sowie Mischoxide zwischen Aluminium und weiteren Metallen, insbesondere zwischen Aluminium und Titan, wie etwa Aluminiumtitanat oder Aluminiumtitanoxid, wobei Aluminiumhydroxid beinhaltende Aluminiumverbindungen, insbesondere die unter der Bezeichnung Alhydrogel^{®} erhältlichen Aluminiummetahydroxide am meisten bevorzugt sind. Diese Aluminiummetahydroxide sind kommerziell in kristalliner Form erhältlich. Denkbar sind weiterhin Aluminiumverbindungen beinhaltend Mischungen aus mindestens zwei der vorstehend genannten Verbindungen, beispielsweise Mischungen beinhaltend Aluminiumphosphat und Aluminiumhydroxid, insbesondere Mischungen beinhaltend Aluminiumhydroxyphosphat-Gel und Al(O)OH.

Weiterhin ist es erfindungsgemäß bevorzugt, dass die Aluminiumverbindung in der erfindungsgemäßen Impfstoffzusammensetzung vorzugsweise in partikulärer Form vorliegt, wobei es in diesem Zusammenhang besonders bevorzugt ist, dass mindestens 75 %, noch mehr bevorzugt mindestens 85 % und am meisten bevorzugt mindestens 95 % der Partikel, jeweils bezogen auf die Teilchenzahl, kleiner sind als 2 µm. Weiterhin ist es in diesem Zusammenhang bevorzugt, dass die Partikel der Aluminiumverbindung vorzugsweise eine Größe in einem Bereich von 0,5 bis 15 µm, besonders bevorzugt von 1 bis 10 µm aufweisen.

Die Oberfläche der im Verfahrensschritt i) bereitgestellten Aluminiumverbindung ist mit Phosphat-Ionen in Kontakt gebracht worden. Dieses erfolgt vorzugsweise dadurch, dass die Aluminiumverbindung in einer wässrigen Phase P₁ gelöst oder dispergiert, vorzugsweise dispergiert wird, welche ein vorzugsweise wasserlösliches Salz eines Phosphates beinhaltet, wobei das Salz eines Phosphates vorzugsweise ausgewählt ist aus der Gruppe umfassend Natriumphosphat, Natriumdihydrogenphosphat, Dinatrium-hydrogenphosphat, Kaliumphosphat, Dikaliumhydrogenphosphat, Kalium-dihydrogenphosphat oder Mischungen aus mindestens zwei dieser Phosphate, wobei Mischungen aus Dinatriumhydrogenphosphat und Natriumdihydrogenphsophat am meisten bevorzugt sind. Erfindungsgemäß insbesondere geeignet ist der Einsatz eines sogenannten PBS-Puffers (PBS = Phosphate Buffered Saline) als wässrige Phase für die Aluminiumverbindung. Dieser PBS-Puffer enthält in seiner normalen Konzentration (= 1×PBS) üblicherweise
- Dinatrium- oder Dikaliumhydrogenphosphat, welches vorzugsweise in einer Konzentration in einem Bereich von 1 bis 10 mM, besonders bevorzugt von 3 bis 8 und am meisten bevorzugt von etwa 5 mM vorliegt,
- Natrium- oder Kaliumdihydrogenphosphat, welches vorzugsweise in einer Konzentration in einem Bereich von 1 bis 10 mM, besonders bevorzugt von 3 bis 8 und am meisten bevorzugt von etwa 5 mM vorliegt, sowie
- NaCl, welches vorzugsweise in einer Konzentration in einem Bereich von 125 bis 175 mM, besonders bevorzugt von 140 bis 160 mM und am meisten bevorzugt in einer Konzentration von etwa 150 mM vorliegt.

Die angegebenen Mengen für die einzelnen Komponenten des PBS-Puffers entsprechen denjenigen Mengen im einfach konzentrierten PBS-Puffer (= 1×fach PBS). Der pH-Wert der wässrigen Phase P₁, insbesondere des PBS-Puffers, liegt bei 25°C vorzugsweise in einem Bereich von 6 bis 8, besonders bevorzugt von 6,5 bis 7,5.

Gemäß einer besonders bevorzugten Ausführungsform des erfindungsgemäßen Verfahrens zur Herstellung einer Impfstoffzusammensetzung wird als wässrige Phase P₁, in der die Aluminiumverbindung gelöst oder dispergiert, vorzugsweise dispergiert wird, ein 1,5× bis 6×PBS-Puffer, besonders bevorzugt ein 1,75× bis 5×PBS-Puffer und am meisten bevorzugt ein 2× bis 4×PBS-Puffer eingesetzt, wobei ein 2× bis 4×PBS-Puffer die vorstehend genannten Konzentrationen an Natriumchlorid, Dinatrium- oder Dikaliumhydrogenphosphat und Natrium- oder Kaliumdihydrogenphosphat in der 2fachen bis 4fachen Menge derjenigen Konzentrationen enthält, die vorstehend als bevorzugte, besonders bevorzugte und am meisten bevorzugte Konzentration für die einzelnen Komponenten angegeben wurden. So enthält beispielsweise ein 1,5×PBS-Puffer
- Dinatrium- oder Dikaliumhydrogenphosphat vorzugsweise in einer Konzentration in einem Bereich von 1,5 bis 15 mM, besonders bevorzugt von 4,5 bis 12 und am meisten bevorzugt von etwa 7,5 mM,
- Natrium- oder Kaliumdihydrogenphosphat vorzugsweise in einer Konzentration in einem Bereich von 1,5 bis 15 mM, besonders bevorzugt von 4,5 bis 12 und am meisten bevorzugt von etwa 7,5 mM, und
- NaCl vorzugsweise in einer Konzentration in einem Bereich von 187,5 bis 262,5 mM, besonders bevorzugt von 210 bis 240 mM und am meisten bevorzugt in einer Konzentration von etwa 225 mM.

Gemäß der am meisten bevorzugten Ausführungsform des erfindungsgemäßen Verfahrens wird im Verfahrensschritt i) ein 2× bis 4×PBS-Puffer vorgelegt, in dem ein Aluminiummetahydroxid, insbesondere die unter der Bezeichnung Al-hydrogel^{®} erhältliche Aluminiumverbindung dispergiert ist.

Sofern im erfindungsgemäßen Verfahren zur Herstellung einer Impfstoffzusammensetzung die Bedingung a) erfüllt ist, wird die Aluminiumverbindung nach dem in Kontakt bringen mit den Phosphat-Ionen und vor dem Verfahrensschritt iii) erhitzt, wobei dieses Erhitzen vorzugsweise auf eine Temperatur von mindestens 100°C, vorzugsweise von mindestens 110°C und am meisten bevorzugt von mindestens 120°C, vorzugsweise auf eine Temperatur in einem Bereich von 100 bis 150°C, besonders bevorzugt auf eine Temperatur in einem Bereich von 110 bis 140°C und am meisten bevorzugt auf eine Temperatur in einem Bereich von 120 bis 130°C erfolgt. Die Dauer des Erhitzens auf eine Temperatur innerhalb der vorstehend genannten Temperaturbereiche liegt üblicherweise bei mindestens 10 Minuten, besonders bevorzugt bei mindestens 15 Minuten und am meisten bevorzugt bei Mindestens 20 Minuten, wobei vorteilhafterweise eine Dauer von 4 Stunden, besonders bevorzugt von 2 Stunden nicht überschritten wird. Weiterhin kann es vorteilhaft sein, die wässrige Phase P₁ während des Erhitzens kontinuierlich zu rühren, um ein möglichst homogenes in Kontakt bringen der Oberfläche der Aluminiumverbindung mit den Phosphat-Ionen zu gewährleisten. Im Anschluss an das Erhitzen lässt man die heiße, wässrige Phase P₁ vorzugsweise auf eine Temperatur in einem Bereich von 15 bis 40°C, besonders bevorzugt auf eine Temperatur in einem Bereich von 18 bis 24°C abkühlen.

Wenn als wässrige Phase P₁, in der die Aluminiumverbindung gelöst bzw. dispergiert ist, eine konzentrierte PBS-Lösung, wie vorstehend beschrieben, eingesetzt worden ist, kann sich an das Erhitzen dieser PBS-Lösung noch ein Verdünnungsschritt anschließend, in dem die konzentrierte PBS-Lösung mit entionisiertem oder destilliertem Wasser gegebenenfalls verdünnt wird.

Sofern im erfindungsgemäßen Verfahren zur Herstellung einer Impfstoffzusammensetzung die Bedingung b) erfüllt ist, beinhaltet die wässrige Phase P₁ nach dem Erhitzen, sofern dieses durchgeführt wird, und insbesondere nach einem gegebenenfalls durchgeführten Verdünnungsschritt weniger als 10 mg/ml Aluminium, besonders bevorzugt weniger als 5 mg/ml, darüber hinaus noch mehr bevorzugt weniger als 2,5 mg/ml und am meisten bevorzugt weniger als 1 mg/ml der Aluminiumverbindung.

Im Verfahrensschritt ii) des erfindungsgemäßen Verfahrens, der vor, während oder auch nach dem Verfahrensschritt i) durchgeführt werden kann, wird ein Volumen V₂ einer wässrigen Phase P₂ beinhaltend ein Antigen bereitgestellt. Auch bei der wässrigen Phase P₂ kann es sich, je nach dem enthaltenen Antigen, um eine wässrige Lösung, um eine wässrige Dispersion, um eine wässrige Emulsion oder um eine wässrige Suspension handeln.

Bevorzugte Antigene sind ausgewählt aus der Gruppe beinhaltend Antigene oder Antigenzusammensetzungen aus dem HIV-1-Virus, beispielsweise tat, nef, gp120 oder gp160), Antigene oder Antigenzusammensetzungen aus dem humanen Herpes-Virus, beispielsweise gD oder Derivate davon, sogenannte Immediate Early Proteine wie etwa ICP27 aus HSV-1 oder HSV-2, Antigene oder Antigenzusammensetzungen oder Antigenzusammensetzungen aus dem Cytomegalo-Virus, wie etwa gB oder Derivative davon, Antigene oder Antigenzusammensetzungen aus dem Rota-Virus, Antigene oder Antigenzusammensetzungen aus dem Epstein-Barr-Virus, wie etwa gp350 oder Derivative davon, Antigene oder Antigenzusammensetzungen aus dem Varicella-Zoster-Virus, wie etwa gpI, gpII oder IE63), Antigene oder Antigenzusammensetzungen aus Hepatitis-Viren, insbesondere aus dem Hepatitis B-Virus, wie etwa das Hepatitis B Oberflächenantigen (HBsAg), das Hepatitis B Coreantigen (HBcAg) oder Derivate dieser Antigene, aus dem Hepatitis A-Virus, aus dem Hepatitis C-Virus oder aus dem Hepatitis E-Virus, Antigene oder Antigenzusammensetzungen anderer viraler Pathogene, wie etwa Paramyxoviren, Respiratory-Syncytial-Virus, wie etwa dem F- oder G-Proteinen oder deren Derivaten, dem Parainfluenza-Virus, dem Masern-Virus, dem Mumps-Virus, dem humanen Papilloma-Viruses, beispielsweise HPV6, HPV11, HPV16 oder HPV18, den Flavi-Viren, beispielsweise dem Gelbfieber-Virus, dem Dengue-Virus, dem Tick-borne-Encephalitis-Virus oder dem Japanischen Encephalitis-Virus, oder dem Influenza-Virus, Antigene oder Antigenzusammensetzungen aus bakteriellen Pathogenen, beispielsweise *Neisseria spp.,* insbesondere *N. gohorrhea* und *N. meningitidis, Streptococcus spp.,* insbesondere *S*. *pneumonie, S. pyogenes, S. agalactiae, S. mutans, Haemophilus spp.,* beispielsweise *H. influenzae npe B,* nicht typisierbare *H. influenzae, H. ducreyi, Moraxella spp.,* insbesondere *M. catarrhalis,* auch bekannt als *Branhamella catarrhalis, Bordetella spp.,* insbesondere *B. pertussis* (beispielsweise Pertactin, Pertussis-Toxin oder Derivate davon), *B. parapertussis* und *B. bronchiseptica, Mycobacterium spp.,* insbesondere *M. tuberculosis, M. bonis, M. leprae, M. avium, M. paratuberculosis, M. smegmatis, Legionella spp.,* insbesondere *L. pneumophila, Escherichia spp.,* insbesondere enterotoxische *E. coli,* enterohemorragische *E. coli* oder enteropathogene *E. coli, Vibrio spp.,* insbesondere *V. cholera* (beispielsweise das Cholera-Toxin oder Derivate davon) *Shigella spp.,* insbesondere *S. sonnei, S. dysenteriae* oder *S. flexnerii, Yersinia spp.,* insbesondere *Y*. *enterocolitica, Y. pestis, Y. pseudotuberculosis, Campylobacter spp.,* insbesondere *C. jejuni* oder *C. coli, Salmonella spp.,* insbesondere *S*. *typhi, S. paratyphi, S. choleraesuis* oder *S. enteritidis, Listeria spp.,* insbesondere *L. monocytogenes; Helicobacter spp.,* insbesondere *H*. *pylori, Pseudomonas spp.,* insbesondere *P. aeruginosa, Staphylococcus spp.,* insbesondere *S*. *aureus* oder *S*. *epidermidis, Enterococcus spp.,* insbesondere *E. faecalis* oder *E. faecium, Clostridium spp.,* insbesondere *C*. *tetani* (beispielsweise das *Tetanus-Toxin* und dessen Derivate), *C*. *botulinum* (beispielsweise das *Botulinum-Toxin* oder dessen Derivate), *C*. *difficile* (beispielsweise das Clostridium-Toxin A oder B oder deren Derivate), *Bacillus spp.,* insbesondere *B. anthracis, Corynebacterium spp.,* insbesondere *C*. *diphtheriae* (beispielsweise das Diphtherie-Toxin und dessen Derivate), *Borrelia spp.,* insbesondere *B. burgdorferi, B. garinii, B. afzelii, B. andersonii* oder *B. hermsii, Ehrlichia spp.,* insbesondere *E. equi, Rickettsia spp.,* insbesondere *R. rickettsii, Chlamydia spp.,* insbesondere *C*. *trachomatis, C. pneumoniae* oder *C*. *psittaci, Leptospira spp.,* insbesondere *L. interrogans, Treponema spp.,* insbesondere *T. pallidum, T. denticola* oder *T. hyodysenteriae,* Antigene oder Antigenzusammensetzungen aus Parasiten wie *Plasmodium spp.,* insbesondere *P. falciparum, Toxoplasma spp.,* insbesondere *T. gondii, Entamoeba spp.,* insbesondere *E. histolytica, Babesia spp.,* insbesondere *B. microti, Trypanosoma spp.,* insbesondere *T. cruzi, Giardia spp.,* insbesondere *G*. *lamblia, Leshmania spp.,* insbesondere *L. major, Pneumocystis spp.,* insbesondere *P. carinii, Trichomonas spp.,* insbesondere *T. vaginalis, Schisostoma spp.,* insbesondere *S. mansoni,* oder Antigene oder Antigenzusammensetzungen aus Hefen, beispielsweise *Candida spp.,* insbesondere *C*. *albicans,* oder *Cryptococcus spp.,* insbesondere *C*. *neoformans.*

Gemäß einer besonders bevorzugten Ausführungsform des erfindungsgemäßen Verfahrens zur Herstellung einer Impfzusammensetzung handelt es sich bei dem Antigen um das HBsAg, um ein Fragment aus diesem Antigen, um eine Variante dieses Antigens oder des Fragmentes dieses Antigens oder um eine Mischung aus mindestens zwei davon. Dabei wird unter der Bezeichnung "Fragment" eines HBsAg's vorzugsweise ein Polypeptid verstanden, bei dem ein Abschnitt von mindestens 25, vorzugsweise mindestens 50 und besonders bevorzugt mindestens 100 Aminosäuren identisch ist mit einem entsprechenden Abschnitt des HBsAg, während unter der Bezeichnung "Variante" des HBsAg's oder des Fragmentes des HBsAg's vorzugsweise ein Polypeptid verstanden wird, bei dem höchstens 10, vorzugsweise höchstens 5 Aminosäuren, besonders bevorzugt höchstens 2 Aminosäuren und am meisten bevorzugt höchstens 1 Aminosäure des HBsAg's oder des Fragmentes des HBsAg's deletiert, insertiert, substituiert oder aber an das C- und/oder N-terminale Ende angefügt, vorzugsweise substituiert sind bzw. ist. Die Bezeichnung "Variante" umfasst auch Fusionspolypeptide, insbesondere Fusionen mit HBcAg (=Hepatitis B-Coreantigen).

Bei dem HBsAg handelt es sich um ein 226-Aminosäuren Protein (p24/gp27 oder S-Protein), das in 20-30 nm Lipoproteinpartikel selbstassembliert, in denen ungefähr 100-150 Untereinheiten durch multiple inter-und intra-molekulare Disulfidbindungen quervernetzt sind. Die HBsAg's können dabei von allen zur Zeit bekannten 8 HBV-Standardgenotypen A, B, C, D, E, F, G und H abgeleitet sein. Diese 8 Standardgenotypen unterscheiden sich in ca. 8 % ihrer Nukleotidsequenz voneinander (siehe Bartholomeusz, Rev. Med. Virol. 13 (2003), 1-14), wobei die Variation vor allem das HBsAg-Gen betreffen. Vorzugsweise weist der HBV Genotyp A die Referenz-Nukleinsäuresequenz Genbank X02763 bzw. der HBV Genotyp A_{afr} die Referenz-Nukleinsäuresequenz gemäß Genbank AF297621 auf. Für den HBV Genotyp Bₐ ist die Referenz-Nukleinsäuresequenz Genbank D00330, für den Genotyp Bⱼ die Referenz-Nukleinsäuresequenz AB073858. Für den HBV Genotyp C ist die Referenz-Nukleinsäuresequenz Genbank AY206389, für den Genotyp Cₐᵤₛ die Referenz-Nukleinsäuresequenz gemäß Genbank AB048704. Für den Genotyp D ist die Referenz-Nukleinsäuresequenz Genbank X02496, für den Genotyp E Genbank X75657, für den Genotyp F Genbank X69798, für den Genotyp G Genbank AF160501 und für den Genotyp H Genbank AY090454.

Der Begriff "HBsAg" umfasst weiterhin neben dem 226-Aminosäuren Protein auch die beiden M- und L-Proteine, die neben dem HBsAg noch das 55-Aminosäuren lange Prä-S2-Peptid (M-Protein) bzw. das 55-Aminosäuren lange Prä-S2-Peptid und das 120-Aminosäuren lange Prä-S1-Peptid (L-Protein) umfassen.

Gemäß einer bevorzugten Ausführungsform des erfindungsgemäßen Verfahrens beinhaltet die wässrige Phase P₂ nicht nur ein HBsAg, sondern, wie dies in der WO-A-2005/02397 beschrieben ist, wenigstens zwei HBsAg's oder Fragmente oder Varianten davon, wobei sich die HBsAg's im HBV-Genotyp in der S-Region und/oder der Prä-S1-Region unterscheiden.

Das HBsAg bzw. die HBsAg's können entweder aus dem Serum von Patienten mit chronischer Hepatitis B, auf synthetischem Wege oder als rekombinante Polypeptide durch dem Fachmann bekannte Verfahren erhalten werden.

Eine Möglichkeit zur Herstellung von HBsAg als rekombinantes Polypeptid besteht darin, das core- bzw. pre-core-Leseraster mittels Polymerasekettenreaktion (PCR) aus einem Virusisolat zu amplifizieren. Denkbar ist auch, die entsprechende DNA-Sequenz auf synthetischem Weg herzustellen und anschließend mittels PCR zu amplifizieren. Das erhaltene PCR-Fragment wird dann in einen Plasmidvektor kloniert. Mittels weiterer PCR-Klonierungen kann dann die entsprechend verkürzte Form in einen Expressionsvektor, beispielsweise in einen Expressionsvektor für *E*. *coli,* insertiert werden. Dabei erfolgt die Insertion vorzugsweise dergestalt, dass das für das HBsAg bzw. für das Fragment oder die Variante kodierende Gen unter der Kontrolle eines aktiven Promoters steht. Der Expressionsvektor kann gleichzeitig Induktionselemente zur Erhöhung der Expressionsrate enthalten. Nach der Transformation dieses Expressionsvektors in *E*. *coli* werden Einzelklone erhalten, welche das HBsAg bzw. das Fragment oder die Variante exprimieren. Um die als Antigene dienenden Polypeptide zu erhalten, wird ein Einzelklon in Kulturmedium angeimpft und über Nacht bei 37°C im Schüttelkolben kultiviert. Die *E. coli*-Kultur wird dann zunächst durch Zentrifugation abgeerntet. Das Bakterienpellet wird in Pufferlösung resuspendiert und anschließend entweder durch Ultraschallbehandlung oder durch Hochdruckhomogenisation aufgeschlossen. Das im Homogenisat befindliche HBsAg bzw. HBsAg-Variante wird durch Fällung mit Ammoniumsulfat konzentriert und anschließend durch Gelfiltration von bakteriellen Wirtskomponenten gereinigt.

Grundsätzlich können die in der erfindungsgemäßen Zusammensetzung enthaltenen HBsAg's bzw. deren Fragmente oder Varianten auch auf synthetischem Weg erhalten werden.

Weitere Hinweise zu den Techniken der rekombinanten oder synthetischen Herstellung von Polypeptiden können
- Sambrook, "Molecular Cloning: A Laboratory Manual", Zweite Auflage (1989);
- *"*DNA Cloning", Volumes I Und II (D. N. Glover, Herausgeber., 1985);
- *"*Oligonucleotide Synthesis" (M. J. Gait, Herausgeber, 1986);
- *"*Nucleic Acid Hybridization" (B. D. Hames & S. J. Higgins, Herausgeber, 1984);
- *"*Transcription and Translation" (B. D. Hames & S. J. Higgins, Herausgeber, 1984);
- *"*Animal Cell Culture" (R. I. Freshney, Herausgeber, 1986);
- *"*Immobilized Cells and Enzymes" (IRL Press, 1986);
- B. Perbal, "A Practical Guide to Molecular Cloning" (1984);
- *"*The Methods in Enzymology series" (Academic Press, Inc.), insbesondere Volumes 154 und 155;
- *"*Gene Transfer Vectors for Mammalian Cells" (J. H. Miller und M. P. Calos, Herausgeber, 1987, Cold Spring Harbor Laboratory);
- Mayer und Walker, Herausgeber, (1987), "Immunochemical Methods in Cell and Molecular Biology" (Academic Press, London);
- Scopes, (1987), "Protein Purification: Principles and Practice", Zweite Auflage (Springer-Verlag, N.Y.) und
- *"*Handbook of Experimental Immunology", Volumes I-IV (D. M. Weir und C. C. Blackwell, Herausgeber, 1986)
entnommen werden.

Detailliert beschrieben ist die Herstellung von HBsAg auch in: Brocke P, Schaefer S, Melber K, Jenzelewski V, Müller F, Dahlems U., Bartelsen O., Park KN, Janowicz ZA, Gellissen G: "Recombinant hepatitis B vaccines: disease characterization and vaccine production" (2005) in Gellissen G (Herausgeber): "Production of recombinant proteins - novel microbial and eucaryontic expression systems", Seiten 319-360, Wiley-VCH, Weinheim).

Schließlich kann HBsAg auch kommerziell erworben werden, beispielsweise von der Firma Rhein Biotech GmbH, Düsseldorf.

Weiterhin ist es erfindungsgemäß bevorzugt, dass auch die wässrige Phase P₂ durch ein geeignetes Puffersystem gepuffert ist, beispielsweise durch den im Zusammenhang mit der wässrigen Phase P₁ beschriebenen PBS-Puffer, wobei im Gegensatz zur wässrigen Phase P₁ die wässrige Phase P₂ beinhaltend das Antigen vorzugsweise als 1PBS-Puffer vorliegt.

Zur Herstellung der wässrigen Phase P₂ wird vorzugsweise zunächst eine Antigen-Stammlösung bereitgestellt, welche dann mit einer Pufferlösung, vorzugsweise mit einem 1×PBS-Puffer, verdünnt wird. Die so erhaltene Lösung kann anschließend, bevor sie im Verfahrensschritt iii) zur wässrigen Phase P₁ zugegeben wird, sterilisiert werden, wobei dieses Sterilisieren vorzugsweise durch Sterilfiltration erfolgt.

Sofern im erfindungsgemäßen Verfahren zur Herstellung einer Impfstoffzusammensetzung die Bedingung c) erfüllt ist, beinhaltet die wässrige Phase P₂ weniger als 2 mg/ml, vorzugsweise weniger als 1 mg/ml, besonders bevorzugt weniger als 0,5 mg/ml, darüber hinaus noch mehr bevorzugt weniger als 0,3 mg/ml und am meisten bevorzugt weniger als 0,2 mg/ml des Antigens.

Im Verfahrensschritt iii) des erfindungsgemäßen Verfahrens zur Herstellung einer Impfstoffzusammensetzung wird nun das Volumens V₂ der wässrigen Phase P₂ zum Volumen V₁ der wässrigen Phase P₁ unter Rühren des Volumens V₁ zugegeben, wobei vorzugsweise zunächst das Volumen V₁ der wässrigen Phase P₁ unter Rühren vorgelegt und dann sukzessive das Volumen V₂ der wässrigen Phase P₂ in das Volumen V₁ der wässrigen Phase P₁ eingerührt wird. Gemäß einer besonders bevorzugten Ausführungsform des erfindungsgemäßen Verfahrens zur Herstellung einer Impfzusammensetzung, bei der Aluminiummetahydroxid als Aluminiumverbindung eingesetzt wird, wird zunächst, wie vorstehend beschrieben, das Aluminiummetahydroxid in einem 1,5× bis 6×PBS-Puffer, besonders bevorzugt in einem 1,75× bis 5×PBS-Puffer und am meisten bevorzugt in einem 2× bis 4×PBS-Puffer unter Rühren vorgelegt, dann unter Rühren auf die vorstehend beschriebene Temperatur und für die vorstehend beschriebene Dauer erhitzt, danach auf die vorstehend beschriebene Temperatur abgekühlt, mit Wasser gegebenenfalls verdünnt und in das so erhaltene Volumen V₁ der wässrigen Phase P₁ das Volumen V₂ der wässrigen Phase P₂ eingerührt.

Sofern im erfindungsgemäßen Verfahren zur Herstellung einer Impfstoffzuammensetzung die Bedingung d) erfüllt ist, wird das Volumen V₂ mit einer Geschwindigkeit von höchstens 10 %, besonders bevorzugt von höchstens 5 %, darüber hinaus bevorzugt von höchstens 2,5 % und am meisten bevorzugt von höchstens 1 % des Betrages des Volumens V₁/Minute zum Volumen V₁ zugegeben. Wird beispielsweise eine wässrige Phase P₁ mit einem Volumen von 20 Litern vorgelegt, so wird das Volumen V₂ mit einer Geschwindigkeit von höchstens 2 Liter/Minute, besonders bevorzugt von höchstens 1 Liter/Minute, darüber hinaus bevorzugt von höchstens 0,5 Liter/Minute und am meisten bevorzugt von höchstens 0,2 Liter/Minute zugesetzt, wobei das Zusetzen des Volumens V₂ zum Volumen V₁ vorzugsweise unter kontinuierlichem Rühren des Volumens V₁ erfolgt.

Weiterhin ist es erfindungsgemäß bevorzugt, dass der Betrag des Volumens V₂ etwa 10 bis 50 %, vorzugsweise etwa 15 bis 40 % und am meisten bevorzugt etwa 20 bis 30 % des Betrages des Volumens V₁ beträgt.

Durch den Einsatz sehr verdünnter Lösungen der Aluminiumverbindung und des Antigens und durch die gezielt langsame Zugabe der wässrigen Phase P₂ des Antigens zur wässrigen Phase P₁ der Aluminiumverbindung wird eine sehr homogene Immobilisierung des Antigens auf der Oberfläche der Aluminiumverbindung erzielt. Diese homogene Verteilung ist unter anderem verantwortlich für die vorteilhaften Eigenschaften der erfindungsgemäßen Impfstoffzusammensetzung, insbesondere für die gute Stabilität sowie die Konstanz der immunstimulatorischen Wirkung in unterschiedlichen Chargen der Impfstoffzusammensetzung.

Gemäß einer besonderen Ausführungsform des erfindungsgemäßen Verfahrens zur Herstellung einer Impfstoffzusammensetzung umfasst das Verfahren zusätzlich die folgenden Verfahrensschritte:
iv) Bereitstellen eines Volumens V₃ einer wässrigen Phase P₃ eines von der Aluminiumverbindung verschiedenen Adjuvants,
v) Zugabe des Volumens V₃ der wässrigen Phase P₃ zum Volumen V₁ der wässrigen Phase P₁, zum Volumen V₂ der wässrigen Phase P₂ oder zu der im Verfahrensschritt iii) erhaltenen Mischung aus den Volumen V₁ und V₂, vorzugsweise jedoch zu der im Verfahrensschritt iii) erhaltenen Mischung aus den Volumen V₁ und V₂,
wobei mindestens eine, vorzugsweise jede der folgenden Bedingungen erfüllt ist:
e) die wässrige Phase P₃ beinhaltet weniger als 1 mg/ml des Adjuvants;
f) das Volumen V₃ wird mit einer Geschwindigkeit von höchstens 10 % des Betrages des Volumens V₁/Minute zum Volumen V₁, von höchstens 10 % des Betrages des Volumens V₂/Minute zum Volumen V₂ bzw. von höchstens 10 % des Betrages des Volumens der Mischung aus den Volumen V₁ und V₂/Minute zur Mischung aus den Volumen V₁ und V₂, vorzugsweise jedoch von höchstens 10 % des Betrages des Volumens der Mischung aus den Volumen V₁ und V₂/Minute zur Mischung aus den Volumen V₁ und V₂, zugegeben.

Erfindungsgemäß bevorzugte Ausführungsformen dieser wiederum bevorzugten Ausführungsform des erfindungsgemäßen Verfahrens sind diejenigen Verfahren, bei denen die folgenden Eigenschaften bzw. Kombinationen von Eigenschaften erfüllt sind: a)e), a)f), b)e), b)f), c)e), c)f), d)e), d)f), a)e)f), b)c)d)e)f) und a)b)c)d)e)f), wobei a), b)c)d)e)f) und a)b)c)d)e)f) besonders bevorzugt und a)b)c)d)e)f) am meisten bevorzugt ist.

Im Verfahrensschritt iv) dieser besonderen Ausführungsform des erfindungsgemäßen Verfahrens zur Herstellung einer Impfstoffzusammensetzung wird zunächst ein Volumen V₃ einer wässrigen Phase P₃ eines von der Aluminiumverbindung verschiedenen Adjuvants bereitgestellt, wobei es sich bei der wässrigen Phase P₃ ebenso wie bei den wässrigen Phasen P₁ und P₂ um eine wässrige Lösung, um eine wässrige Dispersion, um eine wässrige Emulsion oder um eine wässrige Suspension handeln kann.

Bei dem Adjuvants, welches in der wässrigen Lösung enthalten ist, kann es sich um jedes dem Fachmann bekannte Adjuvants handeln, welches üblicherweise in Impfstoffzusammensetzung eingesetzt wird. Allgemein wird dabei unter dem Begriff "Adjuvans" eine Verbindung verstanden, welche die Induktion einer Immunantwort, vorzugsweise die Induktion einer zellulären Immunantwort auf das in der wässrigen Phase P₂ enthaltene Antigen ermöglicht.

Erfindungsgemäß bevorzugte, von Aluminiumverbindungen verschiedene Adjuvantien sind insbesondere ausgewählt aus der Gruppe umfassend aluminiumfreie, partikuläre Adjuvantien, wie beispielsweise Submikro-Öl-in-Wasser-Emulsion, zu denen etwa MF59 (5 % Squalen, 0,5 % Tween 80 und 0,5 % Span 85), SAF (10 % Squalen, 0,4 % Tween 80, 5 % Pluron-blockiertes Polymer sowie thr-Muramyldipeptid (thr-MDP) gehören, unvollständiges Freundsches Adjuvans, Lipopolysaccharide, N-Acetylglucosaminyl-N-acetylmuramyl-L-alanyl-Dipeptid (GMDP) oder Muramyldipeptid (MDP), GAL-Ceramid, Dimethyldioctadecylammoniumbromid (DDAB), Liposomen, vorzugsweise unfassend Phospholipid und Cholesterin, Mikropartikel aus biologisch abbaubaren Polymeren wie Poly-Lactid-co-glykolid (PLGA), Oligodesoxyribonukleotide mit oder ohne CpG-Motiv, N,N-Di-(β-Stearoylethyl)-N,N-dimethylammonium-chlorid (EQ1), Glykopeptide, Bestandteile der Zellwand von Mycobakterien, quaternäre Amine wie beispielsweise Cetylpyridiniumchlorid und Benzalkoniumchlorid, zwitterionische Amine wie CHAPS (3-(3-Cholamidopropyl)-dimethyl-ammonio)-1-propansulfonat), Dextransulfat, Granulozyten-Makrophagen-Kolonien stimulierender Faktor (GM-CSF), Tumornekrosefaktor (TNF), , Blockcopolymere wie beispielsweise P1205 oder Poloxamer 401, Dimyristoylphosphatidylcholin (DMPC), 3β-Hydroxy-5-androsten-17-on (DHEA), Dimyristoylphosphatidylglycerol (DMPG), Desoxycholsäure-Natriumsalz, Imiquimod, DTP-GDP, 7-Allyl-8-Oxoguanosin, Montanide ISA 51, Montanide ISA 720, Murametid, Murapalmitin, Dicetylphosphat, Polymethylmethacrylat (PMMA), PEG-Sorbitanfettsäureester wie Polysorbat 20 oder80 (TWEEN 20,80), detoxifizierte Mutanten von bakteriellen ADP-ribosylierenden Toxinen wie Cholera-Toxin oder Pertussis-Toxin oder Mischungen aus mindestens zwei dieser Adjuvantien. Als Co-Adjuvantien besonders bevorzugt sind Lipopolysaccharide, Aluminiumhydroxid, Aluminiumphosphat, Gal-Ceramid, Oligodesoxyribonukleotide mit CpG-Motiv, PEG-Sorbitanfettsäureester, ein Saponin oder ein Saponinderivat beinhaltende Adjuvantien, wie etwa die kommerziell erhältlichen Produkte AbISCO^{®}-100, AbISCO^{®}-200, AbISCO^{®}-300 (ISCONOVA, Uppsala, Schweden) oder Stimulon^{®}QS-21 (ANTIGENICS, Woburn, USA), sowie 3-O-deacetyliertes Monophosphoryl-Lipid A.

Eine Übersicht über als Adjuvantien geeignete Verbindungen ist in Cox, J.C. und Coulter, A. R., "Adjuvants-a classification and review of their modes of action" in Vaccine 15:248-256 (Febr. 1997) oder aber auch in Frederick R. Vogel et al., "A Compendium of Vaccine Adjuvants and Excipients", 2. Ausgabe, dargestellt. Die Offenbarung dieser Veröffentlichungen hinsichtlich möglicher Adjuvantien in Impfstoffzusammensetzungen wird hiermit als Referenz eingeführt und bildet einen Teil der Offenbarung der vorliegenden Erfindung.

Auch im Zusammenhang mit der wässrigen Phase P₃ beinhaltend das Adjuvants kann es erfindungsgemäß vorteilhaft sein, dass diese durch ein geeignetes Puffersystem gepuffert ist, beispielsweise durch den im Zusammenhang mit den wässrigen Phasen P₁ und P₂ beschriebenen PBS-Puffer, wobei im Gegensatz zur wässrigen Phase P₁ und in Übereinstimmung mit der wässrigen Phase P₂ die wässrige Phase P₃ beinhaltend das Adjuvants vorzugsweise als 1×PBS-Puffer vorliegt. Insbesondere bei der Verwendung der Derivate eines 3-O-deacetylierten Monophosphoryl-Lipids A als Adjuvants ist es jedoch bevorzugt, entionisiertes oder destilliertes Wasser als Lösungsmittel einzusetzen.

Zur Herstellung der wässrigen Phase P₃ wird vorzugsweise zunächst eine Adjuvants-Stammlösung bereitgestellt, welche dann mit einer Pufferlösung oder mit entionisiertem oder destilliertem Wasser, vorzugsweise mit entionisiertem oder destilliertem Wasser, verdünnt wird.

Die so erhaltene wässrige Phase P₃ kann anschließend sterilisiert werden, wobei dieses Sterilisieren vorzugsweise durch Sterilfiltration erfolgt.

Sofern bei dieser besonders bevorzugten Ausführungsform des erfindungsgemäßen Verfahrens zur Herstellung einer Impfstoff-zusammensetzung die Bedingung e) erfüllt ist, beinhaltet die wässrige Phase P₃ weniger als 1 mg/ml, vorzugsweise weniger als 0,75 mg/ml, besonders bevorzugt weniger als 0,5 mg/ml, darüber hinaus noch mehr bevorzugt weniger als 0,3 mg/ml und am meisten bevorzugt weniger als 0,15 mg/ml des Adjuvants.

Im Verfahrensschritt v) dieser besonderen Ausführungsform des erfindungsgemä-ßen Verfahrens zur Herstellung einer Impfstoffzusammensetzung wird nun das Volumen V₃ der wässrigen Phase P₃ zum Volumen V₁ der wässrigen Phase P₁, zum Volumen V₂ der wässrigen Phase P₂ oder zu der im Verfahrensschritt iii) erhaltenen Mischung aus den Volumen V₁ und V₂, vorzugsweise jedoch zu der im Verfahrensschritt iii) erhaltenen Mischung aus den Volumen V₁ und V₂, zugegeben.

Sofern bei dieser besonderen Ausführungsform des erfindungsgemäßen Verfahrens zur Herstellung einer Impfstoffzuammensetzung die Bedingung f) erfüllt ist, wird das Volumen V₃ wird mit einer Geschwindigkeit von höchstens 10 %, besonders bevorzugt von höchstens 5 %, noch mehr bevorzugt von höchstens 2,5 % und am meisten bevorzugt von höchstens 1 % des Betrages des Volumens V₁, des Betrages des Volumens V₂ oder des Betrages der Mischung aus den Volumen V₁ und V₂/Minute zum Volumen V₁, zum Volumen V₂ bzw. zur Mischung aus den Volumen V₁ und V₂ zugegeben.

Weiterhin ist es bei dieser besonderen Ausführungsform des erfindungsgemäßen Verfahrens bevorzugt, dass das Volumen V₃ der wässrigen Phase P₃ etwa 20 bis 70 %, besonders bevorzugt etwa 30 bis 60 % und am meisten bevorzugt etwa 40 bis 50 % des Volumens V₁ der wässrigen Phase P₁ beträgt.

Schließlich kann das erfindungsgemäße Verfahren auch noch weitere Verfahrensschritte umfassen, insbesondere einen Verfahrensschritt vi), in dem dem Volumen V₁, dem Volumen V₂, dem Volumen V₃, der Mischung aus den Volumen V₁ und V₂, der Mischung aus den Volumen V₁ und V₃, der Mischung aus den Volumen V₂ und V₃ oder der Mischung aus den Volumen V₁, V₂ und V₃, besonders bevorzugt jedoch der Mischung aus den Volumen V₁, V₂ und V₃ ein weiteres Volumen V₄ einer vorzugsweise wässrigen und vorzugsweise sterilen Phase P₄ beinhaltend einen oder mehrere Zusatzstoffe, beispielsweise Antioxidationsmittel, Pufferkomponenten, Farbstoffe, Viskositätsregulierer oder Konservierungsmittel, zugesetzt wird.

Einen Beitrag zur Lösung der eingangs genannten Aufgaben leistet auch eine Impfstoffzusammensetzung, erhältlich durch das vorstehend beschriebene, erfindungsgemäße Verfahren.

Einen weiteren Beitrag zur Lösung der eingangs genannten Aufgaben leistet auch eine Impfstoffzusammensetzung, beinhaltend
I) ein Substrat umfassend eine Aluminiumverbindung, deren Oberfläche mit Phosphat-Anionen in Kontakt gebracht worden ist, sowie
II) ein Antigen, welches auf dem Substrat immobilisiert ist,
   wobei das Antigen homogen auf dem Substrat immobilisiert ist.
   Als Aluminiumverbindung und als Antigen sind dabei diejenigen Verbindungen bzw. Komponenten bevorzugt, die bereits eingangs im Zusammenhang mit dem erfindungsgemäße Verfahren zur Herstellung einer Impfstoffzusammensetzung als bevorzugte Antigene bzw. Aluminiumverbindungen genannt worden sind.
   Unter einer "homogenen" Immobilisierung des Antigens auf dem Substrat wird dabei vorzugsweise eine Immobilisierung verstanden, die bei einer immunhistochemischen Anfärbung der erfindungsgemäßen Impfstoffzusammensetzung mittels FITC- oder TRITC-markierter und gegen das jeweilige Antigen gerichteter monoklonaler Antikörper eine homogen Färbung der Substratoberfläche erkennen lassen.
   Gemäß einer besonderen Ausführungsform der erfindungsgemäßen Impfstoffzusammensetzung umfasst diese zusätzlich
III) ein Adjuvants, welches ebenfalls auf dem Substrat immobilisiert ist,
wobei das Adjuvants ebenfalls homogen auf dem Substrat immobilisiert ist. Als Adjuvants sind ebenfalls diejenigen Verbindungen oder Komponenten bevorzugt, die bereits eingangs im Zusammenhang mit dem erfindungsgemäßen Verfahren zur Herstellung einer Impfstoffzusammensetzung als bevorzugte Adjuvantien beschrieben worden sind.

Einen weiteren Beitrag zur Lösung der eingangs genannten Aufgaben leistet auch die Verwendung der durch das erfindungsgemäße Verfahren erhältlichen Impfstoffzusammensetzung oder der erfindungsgemäßen Impfstoffzusammensetzung zur Herstellung eines Arzneimittels zur Prophylaxe oder Therapie, vorzugsweise der Prophylaxe, von Viruserkrankungen, insbesondere die Verwendung der Impfstoffzusammensetzung beinhaltend HBsAg als Antigen als Impfstoff zur Prophylaxe oder Therapie, vorzugsweise der Prophylaxe, einer Hepatitis B-Erkrankung beim Menschen.

Der Begriff "Prophylaxe" im Zusammenhang mit einer HBV-Infektion beim Menschen umfasst dabei vorzugsweise
- die Behandlung eines Menschen, welcher noch keinen Kontakt mit HBV hatte, mit dem Ziel, zu verhindern, dass eine Infektion mit HBV erfolgt,
- die Behandlung eines Menschen, welcher bereits Kontakt mit HBV hatte und der sich in der immunotoleranten chronischen Phase befindet, mit dem Ziel, zu verhindern, dass dieser Mensch in die immunoaktive chronische Phase eintritt, sowie
- die Behandlung eines Menschen, welcher bereits Kontakt mit HBV hatte und der sich im inaktiven Trägerstadium befindet, mit dem Ziel, zu verhindern, dass dieser Mensch erneut in die immunoaktive Phase eintritt,
wobei am meisten bevorzugt unter dem Begriff "Prophylaxe" die Behandlung eines Menschen verstanden wird, welcher noch keinen Kontakt mit HBV hatte, mit dem Ziel, zu verhindern, dass eine Infektion mit HBV erfolgt.

Mengen, mit denen die vorstehend genannten Ziele erreicht werden, werden als "therapeutisch wirksame Dosen" definiert. Die für den jeweiligen Anwendungsfall therapeutisch wirksame Dosis hängt beispielsweise von der genauen Zusammensetzung der erfindungsgemäßen Impfstoffzusammensetzung bzw. des erfindungsgemäßen Arzneimittels, der Verabreichungsart sowie dem Gewicht und dem allgemeinen Gesundheitszustand des Patienten ab, liegt im Allgemeinen jedoch vorzugsweise in einem Bereich von etwa 0,1 bis 2.000 µg (Gesamtmenge aus Aluminiumverbindung, Antigen und gegebenenfalls Adjuvants) für einen 70 kg schweren Patienten, besonders bevorzugt in einem Bereich von 0,5 bis 1.500 µg, darüber hinaus bevorzugt in einem Bereich von 1 bis 1.000 µg und am meisten bevorzugt in einem Bereich von 10 bis 500 µg, wobei die erfindungsgemäße Impfstoffzusammensetzung vorzugsweise in Form von lediglich zwei Dosen, vorzugsweise verabreicht in einem Abstand von 2 Wochen bis 2 Monaten, besonders bevorzugt in einem Abstand von etwa einem Monat, verabreicht wird.

Die erfindungsgemäße Impfstoffzusammensetzung bzw. das erfindungsgemäße Arzneimittel kann grundsätzlich oral, intranasal, intradermal, subkutan, intramuskulär oder intravenös verabreicht werden.

Einen weiteren Beitrag zur Lösung der eingangs genannten Aufgaben leistet ein Verfahren zur Immunisierung eines Menschen, wobei die durch das erfindungsgemäße Verfahren erhältliche Impfstoffzusammensetzung, die erfindungsgemäßen Impfstoffzusammensetzung oder das Arzneimittel einem Patienten, vorzugsweise einem Menschen, verabreicht wird, vorzugsweise in Form einer ersten und einer zweiten Dosis, wobei der Abstand zwischen der ersten und zweiten Dosis vorzugsweise 2 Wochen bis 2 Monate, besonders bevorzugt etwa einen Monat, beträgt.

Die Erfindung wird nun anhand eines nicht limitierenden Beispiels näher erläutert.

### BEISPIEL

Folgende Phasen werden bereitgestellt:
1. In einem autoklavierbaren Gefäß, welches ausgerüstet ist mit einer Rührvorrichtung, werden etwa 1,5 kg Aluminiummetahydroxid-Gel (Alhydrogel^{®}, Brenntag Biosector, Dänemark) in 3×PBS (enthält etwa 2,94 mg/ml Dinatriumhydrogensulfat-Dihydrat, 2,13 mg/ml Natriumdihydrogensulfat-Dihydrat und 27 mg/ml Natriumchlorid) vorgelegt und unter Rühren für mindestens 20 Minuten auf eine Temperatur von 121°C erhitzt. Nach dem Abkühlen auf Raumtemperatur wird anschließend mit destilliertem Wasser auf eine 1,5×PBS-Konzentration verdünnt. Die Konzentration an Alhydrogel^{®} beträgt nach dem Verdünnen etwa 1 mg/ml. Es wird eine wässrige Phase P₁ beinhaltend Alhydrogel^{®} als Aluminiumverbindung erhalten.
2. HBsAg (Rhein Biotech GmbH, Düsseldorf) wird mit PBS (enthält etwa 0,98 mg/ml Dinatriumhydrogensulfat-Dihydrat, 0,71 mg/ml Natriumdihydrogensulfat-Dihydrat und 9 mg/ml Natriumchlorid) auf eine Konzentration von etwa 0,1 mg/ml verdünnt. Es wird eine wässrige Phase P₂ beinhaltend HBsAg als Antigen erhalten.
3. Zu der im autoklavierbaren Gefäß befindlichen wässrigen Phase P₁ (Gesamtvolumen etwa 18 Liter) wird unter Rühren die wässrige Phase P₂ (Gesamtvolumen etwa 4,4 Liter) mit einer Geschwindigkeit von etwa 0,1 Litern/Minute zugesetzt.

Die so erhaltene Impfstoffzusammensetzung war durch eine ausgezeichnete Immunogenität und Stabilität gekennzeichnet.

## Patentansprüche

1. Ein Verfahren zur Herstellung einer Impfstoffzusammensetzung, beinhaltend die Verfahrensschritte:
i) Bereitstellen eines Volumens V₁ einer wässrigen Phase P₁ beinhaltend eine Aluminiumverbindung, deren Oberfläche mit Phosphat-Ionen in Kontakt gebracht worden ist,
ii) Bereitstellen eines Volumens V₂ einer wässrigen Phase P₂ eines Antigens,
iii) Zugabe des Volumens V₂ der wässrigen Phase P₂ zum Volumen V₁ der wässrigen Phase P₁ unter Rühren des Volumens V₁,
wobei mindestens eine, vorzugsweise jede der folgenden Bedingungen erfüllt ist:
a) die Aluminiumverbindung wurde nach dem in Kontakt bringen mit den Phosphat-Ionen und vor dem Verfahrensschritt iii) erhitzt;
b) die wässrige Phase P₁ beinhaltet weniger als 10 mg/ml Aluminium;
c) die wässrige Phase P₂ beinhaltet weniger als 1 mg/ml des Antigens;
d) das Volumen V₂ wird mit einer Geschwindigkeit von höchstens 10 % des Betrages des Volumens V₁/Minute zum Volumen V₁ zugegeben.

2. Das Verfahren nach Anspruch 1, wobei die Aluminiumverbindung Aluminiumhydroxid beinhaltet.

3. Das Verfahren nach Anspruch 1 oder 2, wobei die wässrige Phase P₁ neben der Aluminiumverbindung das Salz eines Phosphates beinhaltet.

4. Das Verfahren Anspruch 3, wobei das Bereitstellen des Volumens V₁ der wässrige Phase P₁ auch das Erhitzen des Volumens V₁ der wässrigen Phase P₁ auf eine Temperatur von mindestens 100°C umfasst.

5. Das Verfahren nach einem der vorhergehenden Ansprüche, wobei die wässrige Phase P₁ weniger als 1 mg/ml Aluminium beinhaltet.

6. Das Verfahren nach einem der vorhergehenden Ansprüche, wobei das Antigen ein HBsAg ist.

7. Das Verfahren nach einem der vorhergehenden Ansprüche, wobei die wässrige Phase P₂ weniger als 0,2 mg/ml Antigen enthält.

8. Das Verfahren nach einem der vorhergehenden Ansprüche, wobei das Volumen V₂ mit einer Geschwindigkeit von höchstens 1 % des Betrages des Volumens V₁/Minute zum Volumen V₁ zugegeben wird.

9. Das Verfahren nach einem der vorhergehenden Ansprüche, wobei der Betrag des Volumens V₂ etwa 10 bis 50 % des Betrages des Volumens V₁ beträgt.

10. Das Verfahren nach einem der vorhergehenden Ansprüche, wobei das Verfahren zusätzlich die folgenden Verfahrensschritte umfasst:
iv) Bereitstellen eines Volumens V₃ einer wässrigen Phase P₃ beinhaltend ein von der Aluminiumverbindung verschiedenes Adjuvants,
v) Zugabe des Volumens V₃ der wässrigen Phase P₃ zum Volumen V₁ der wässrigen Phase P₁, zum Volumen V₂ der wässrigen Phase P₂ oder zu der im Verfahrensschritt iii) erhaltenen Mischung aus den Volumen V₁ und V₂,
wobei mindestens eine, vorzugsweise jede der folgenden Bedingungen erfüllt ist:
e) die wässrige Phase P₃ beinhaltet weniger als 1 mg/ml des Adjuvants;
f) das Volumen V₃ wird mit einer Geschwindigkeit von höchstens 10 % des Betrages des Volumens V₁/Minute zum Volumen V₁, von höchstens 10 % des Betrages des Volumens V₂/Minute zum Volumen V₂ bzw. von höchstens 10 % des Betrages des Volumens der Mischung aus den Volumen V₁ und V₂/Minute zur Mischung aus den Volumen V₁ und V₂ zugegeben.

11. Verfahren nach Anspruch 10, wobei die wässrige Phase P₃ weniger als 0,15 mg/ml Adjuvants beinhaltet.

12. Verfahren nach einem der vorhergehenden Ansprüche, wobei das Volumen V₃ etwa 30 bis 60 % des Volumens V₁ beträgt.

13. Eine Impfstoffzusammensetzung, erhältlich durch das Verfahren nach einem der vorhergehenden Ansprüche.

14. Eine Impfstoffzusammensetzung, beinhaltend
I) ein Substrat umfassend eine Aluminiumverbindung, deren Oberfläche mit Phosphat-Anionen in Kontakt gebracht worden ist, sowie
II) ein Antigen, welches auf dem Substrat immobilisiert ist,
wobei das Antigen homogen auf dem Substrat immobilisiert ist.

15. Die Impfstoffzusammensetzung nach Anspruch 14, wobei die Aluminiumverbindung Aluminiumhydroxid beinhaltet.

16. Die Impfstoffzusammensetzung nach Anspruch 14 oder 15, wobei das Antigen ein HBsAg ist.

17. Die Impfstoffzusammensetzung nach einem der vorhergehenden Ansprüche, wobei die Zusammensetzung zusätzlich
III) ein Adjuvants, welches ebenfalls auf dem Substrat immobilisiert ist,
umfasst, und wobei das Adjuvants ebenfalls homogen auf dem Substrat immobilisiert ist.

18. Verwendung der Impfstoffzusammensetzung nach einem der Ansprüche 13 bis 17 zur Herstellung eines Arzneimittels zur Prophylaxe von Hepatitis B.
